# EUROPEAN PATENT APPLICATION

(11) **EP 2 259 048 A1**
(43) Date of publication of application: **08.12.2010**
(21) Application number: 09161755.5
(22) Date of filing: 03.06.2009
(51) Int. Cl.: G01N 21/47, A61B 5/00

(54) **Measuring reflectance using waveguide for coupling light to larger volume of sample**

(71) Applicant: Koninklijke Philips Electronics N.V., 5621 BA Eindhoven (NL)
(72) Inventor: The designation of the inventor has not yet been filed
(74) Representative: Damen, Daniel Martijn

(57) **Abstract**

Apparatus for measuring reflectance of a sample has a collector (50, 120) for collecting light reflected from the sample, and a waveguide (20, 100, 300) to couple illuminating light to a surface of the sample through an outcoupling surface (100) of the waveguide. The waveguide diffuses the illuminating light so that light leaving the outcoupling surface is evenly spread. The outcoupling surface surrounds one or more light collection sites used by the collector, and is separated from the collection site or sites. By using such a waveguide, a greater volume of the sample can be illuminated and the measurements can be made less sensitive to local variations in the sample. The even spreading of the light and having the outcoupling surface at least partially surrounding a collecting site, can keep the range of penetration depths better defined, and so improve consistency of measurements.

## Description

### FIELD OF THE INVENTION

This invention relates to apparatus for measuring the reflectance from a sample, corresponding methods, and probes for such apparatus for applications such as, inter alia, minimally invasive or non-invasive glucose monitoring.

### BACKGROUND OF THE INVENTION

Many techniques have been investigated to detect skin analyte(s) concentration non-invasively by means of optical, electrical and/or optoelectronic methods, e.g., non-invasive glucose monitoring. Currently a number of companies are developing instruments for non-invasive glucose measurements based on optical methods. A major problem comes from the varying optical properties of human skin tissue. Although these methods are proven to have sufficient sensitivity for in-vitro and/or ex-vivo glucose quantification, none of the currently existing companies have been successful in bringing a non-invasive device to the market. The main reason is that the accuracy and reliability of recently developed devices is not sufficient to get FDA approval.

Non-invasive measurement is the most desirable method for consumers. But the uncertainty and inaccuracy hampers the acceptance of non-invasive testing. There is a need in the non-invasive glucose-monitoring market to solve the inaccuracy and unreliability problems.

Also methods and instruments have been developed for minimally invasive measurement of physiological parameters in a human or animal body, such as, for example, glucose measurements based on optical methods. These methods make use of a sensor, e.g., a biosensor, implanted beneath the skin which is in contact with subcutaneous fluids. The sensor may include gels, particles, or liquids which are biodegradable. Preferably, the biosensor that is implanted is small in size, and does not require a complicated or painful insertion below the skin.

Typically, in vivo measurements of blood analytes deal with a large number of chemical, physical, and physiological interferences in comparison to in vitro cases. These chemical, physical and physiological interferences include, inter alia, the water and salt concentrations in the skin, the level of melanin in the skin, the temperature of the skin, the movement of the skin, and whether the analyte measurement probe is correctly aligned with the muscle below the skin. This is true for both non-invasive and minimally invasive analyte measurement methods. It is known that measurements performed on human tissue are often corrupted by large variations of the measurement sample that do not relate to the desired target analyte. These disturbing and undesired variations are called interferences to the target analyte. Often times these large variations are due to the chemical, physical and physiological interferences like the ones mentioned in the previous paragraph. One attempt to solve this issue is based on mathematical analyses of the measured data. Typical approaches apply chemometrical methods, (e.g., multivariate analysis), to extract the desired information from an analyte measurement that is corrupted by these various interferences. Basically these chemometrical methods are about performing post-processing of the measure data to filter out the interferences corrupting the target analyte concentration. However, for such complex systems as human skin, a large number of interferences can have a large influence on the measured analyte concentration. Therefore, post-processing chemometric methods become very complex and are prone to large errors. The accuracy and reproducibility of these measurements are generally poor due to the many interferences compared with an in vitro case.

It is known from US patent 7307734 disclosing an integrated optical sensor that uses low coherence interferometry one is capable of determining analyte concentration in skin based on the absorption, scattering and polarization of the light emitted from the integrated optical sensor. The sensor includes one or more light collectors, with each collector having a separation distance from the region where the sample is illuminated by the source. Information about the scattering and absorption coefficients of skin can be obtained by measuring individually the collected backscattered light from a plurality of light collectors that is combined with a reference arm light using a coupler, and the intensity of interference detected by a detector.

WO2006079797 shows non invasive glucose monitoring using an attenuated total reflectance, ("ATR"), prism. The prism has a surface for forming an interface with a body region such as skin. The same surface is used for illuminating and measuring, as it relies on total internal reflection, ("TIR"), with only an evanescent wave penetrating a short distance into the skin. Hollow waveguide collimators and detectors are used for measurement and reference wavelength bands.

### SUMMARY OF THE INVENTION

An object of the invention is to provide apparatus for measuring reflectance from a sample, to corresponding methods, and to probes for such apparatus for applications such as, for example, minimally invasive or non-invasive glucose monitoring. According to a first aspect, the invention provides:

Apparatus for measuring the reflectance of a sample, the apparatus comprising a collector for collecting light reflected from the sample, and a waveguide arranged to couple illuminating light to a surface of the sample through an outcoupling surface of the waveguide, the waveguide being arranged to diffuse the illuminating light so that the light leaving the outcoupling surface is evenly spread, and the outcoupling surface has a pattern which at least partially surrounds one or more light collection sites used by the collector, and is separated from the collection site or sites.

By using such a waveguide, a greater volume of the sample can be illuminated and the measurements can be made less sensitive to local variations in the sample. This is particularly useful for measurements of samples having complex structures such as cells or tissue or skin for example, which may give widely different reflections from localized variations such as hairs. The apparatus may also be used with minimally invasive techniques using a microsensor embedded below the skin.

The even spreading of the light and having the outcoupling surface at least partially surrounding a collecting site can help make better measurements from the large volume of the sample which is illuminated. This larger volume can be kept within a better defined range of distances away from the collecting site. This can help keep the range of penetration depths better defined, and so improve consistency of measurements.

Embodiments of the invention can have many other features added; some such additional features are set out in dependent claims and described in more detail below.

Other aspects of the invention include a probe for such apparatus, and to corresponding methods of making measurements.

Many of the additional features can be combined together and combined with any of the aspects of the invention. Other advantages may be apparent to those skilled in the art, especially over the prior art. Numerous variations and modifications can be made without departing from the claims of the present invention. Therefore, it should be clearly understood that the form of the present invention is illustrative only and is not intended to limit the scope of the present invention.

### BRIEF DESCRIPTION OF THE DRAWINGS

How the present invention may be put into effect will now be described by way of example with reference to the appended drawings, in which:
FIG. 1 shows a 3D perspective view of an apparatus according to a first embodiment.
FIG. 2 shows an apparatus according to another embodiment.
FIG. 3 shows a plan view of another embodiment.
FIG. 4 shows a side cross section view of the embodiment of FIG. 3.
FIG. 5 shows a plan view of another embodiment.
FIG. 6 shows a side cross section view of the embodiment of FIG. 5.
FIG. 7 shows a plan view of another embodiment.
FIG. 8 shows a 3D perspective view of an apparatus according to another embodiment, with an inset showing scattering at the outcoupling surface.
FIG. 9 shows a 3D perspective view of an apparatus according to another embodiment, showing a perpendicular waveguide.
FIGs. 10 and 11 show plan views of other embodiments.
FIG. 12 shows an alternative illuminating arrangement for the embodiment of FIG. 11.

### DETAILED DESCRIPTION SOME EMBODIMENTS

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g., "a" or "an", "the", this includes a plural of that noun unless something else is specifically stated.

The term "comprising", used in the claims, should not be interpreted as being restricted to the means listed thereafter; it does not exclude other elements or steps. Thus, the scope of the expression "a device comprising means A and B" should not be limited to devices consisting only of components A and B. It means that with respect to the present invention, the only relevant components of the device are A and B.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention. References to a signal can encompass any kind of signal in any medium, and so can encompass an electrical or optical or wireless signal or other signal for example. References to analyzing can encompass processing a signal in any way to derive or enhance information about the material. References to a controller can encompass any means for controlling and so can encompass for example a personal computer, a microprocessor, analog circuitry, application specific integrated circuits, and software for the same, and so on.

In the description provided herein, numerous specific details are set forth. However, it is understood that embodiments of the invention may be practiced without these specific details. In other instances, well-known methods, structures and techniques have not been shown in detail in order not to obscure an understanding of this description.

References to a waveguide are intended to encompass one piece or multipiece waveguides, solid or hollow waveguides, using reflecting surfaces or TIR, or any arrangements of reflecting surfaces to achieve a similar effect.

Additionally, the term, and all forms of the term, "sense or sensing" is used synonymously herein with the term, and all forms of the term, "detect" or "detecting".

### Introduction to some issues addressed by some of the embodiments

By way of introduction to the embodiments, the problems of measuring an analyte through the skin, in particular, by minimally invasive and non invasive blood glucose monitoring, will be discussed briefly. The measurement of glucose through near-infrared spectroscopy is based on a change in the concentration of glucose being indicated by a change in the absorption of light according to the absorption and scattering properties of glucose and/or the effect of glucose changes upon the anatomy and physiology of the sampled site. However, in addition to the effect of glucose on the near-infrared light probing signal that is delivered to the skin, the probing signal is also reflected, diffusely reflected, transmitted, scattered, and absorbed in a complex manner related to the structure and composition of the tissue. When near-infrared light is delivered to the skin, a proportion of reflected light, or specular reflectance, is typically between 4-7% of the delivered light over the entire spectrum. Absorption by the various skin constituents accounts for the spectral extinction of the light within each layer. Scattering is the main process by which the beam may be returned to contribute to the diffuse reflectance of the skin. Scattering also has a strong influence on the light that is diffusely transmitted through a portion of the skin.

The scattering of light in tissues is in part due to discontinuities in the refractive indices on the microscopic level, such as the aqueous-lipid membrane interfaces between each tissue compartment or the collagen fibrils within the extracellular matrix. The spectral characteristics of diffuse remittance from tissue result from a complex interplay of the intrinsic absorption and scattering properties of the tissue, the distribution of the heterogeneous scattering components, and the geometry of the point(s) of irradiation relative to the point(s) of light detection.

The near-infrared absorption of light in tissue is primarily due to overtone and combination absorbances of the C-H, N-H, and O-H functional groups. As skin is primarily composed of water, protein, and fat; these functional groups dominate the near-IR absorption in tissue. As the main constituent, water dominates the near-infrared absorbance above 1100 nm and is observed through pronounced absorbance bands at 1450, 1900, and 2600 nm. Protein in its various forms, in particular, collagen is a strong absorber of light that irradiates the dermis. Near-infrared light that penetrates the subcutaneous tissue is absorbed primarily by fat. In the absence of scattering, the absorbance of near-infrared light due to a particular analyte, A, can be approximated by Beer's Law. An approximation of the overall absorbance at a particular wavelength is the sum of the individual absorbance of each particular analyte given by Beer's Law. The concentration of a particular analyte, such as glucose, can be determined through a multivariate analysis of the absorbance over a multiplicity of wavelengths because it is unique for each analyte. However, in tissue compartments expected to contain glucose, the concentration of glucose is at least three orders of magnitude less than that of water. Given the known extinction coefficients of water and glucose, the signal targeted for detection by reported approaches to near-infrared measurement of glucose, i.e. the absorbance due to glucose in the tissue, is expected to be, at most, three orders of magnitude less than other interfering tissue constituents. Therefore, the near-infrared measurement of glucose requires a high level of sensitivity over a broad wavelength range. Multivariate analysis is often utilized to enhance sensitivity, but often this sensitivity is still inadequate for accurate and reliable analyte measurement.

In addition, the diverse scattering characteristics of the skin, e.g., multiple layers and heterogeneity, cause the light returning from an irradiated sample to vary in a highly nonlinear manner with respect to tissue analytes, in particular, glucose. Simple linear models, such as Beer's Law have been reported to be invalid for the dermis.

The measurement of glucose through spectroscopy can also be made by a change in the absorption of light according to the absorption and scattering properties of minimally invasive microsensors, or to the change in light emitted or reflected from such microsensors located below the skin. Such methods using microsensors may include, for example,
- observing fluorescence (e.g., fluorescence resonance energy transfer) of a competitive binding assay encapsulated in microcapsules, for example based on competitive binding between the protein Concanavalin A and various saccharide molecules, specifically a glycodendrimer and glucose, the microcapsules can be polyelectrolyte microcapsules;
- detecting glucose using boronic acid-substituted viologens in fluorescent hydrogels in which a fluorescent anionic dye and a viologen are appended to boronic acid, which serve as glucose receptors, and are immobilized into a hydrogel, the fluorescence of the dye being modulated by the quenching efficiency of the viologen based receptor which is dependent upon the glucose concentration;
- other methods, e.g., monitoring oxygen or pH or other "smart tattoo" related methods.

Dynamic properties of the skin also add to the difficulties. Variations in the physiological state and fluid distribution of tissue profoundly affect the optical properties of tissue layers and compartments over a relatively short period of time. For all these reasons therefore, the optical properties of the tissue sample are modified in a highly nonlinear and profound manner that introduces significant interference into minimally invasive or non-invasive tissue measurements.

### Volume dependence

Most of these non-invasive techniques to measure analytes in the body use a small measurement volume. In minimally invasive methods the microsensor is normally small in size. Using a small measurement volume makes the technique more vulnerable to the structural heterogeneity of the skin. The contribution of small interfering structures such as hairs in the skin is relatively large and leads to a high sensitivity of the technique to accurate repositioning for comparative measurements. Using a large measurement volume to capture the resulting signal can make the measurements more representative of the 'average' skin, and accurate repositioning of the measuring probe less critical. This enhances the accuracy, reproducibility and usability of the sensing device.

### Introduction to features of the embodiments

Embodiments of the invention can have an illuminator for directing light onto the sample. This can be any kind of illuminator and can encompass a light source or can be for directing light received from an external source. The illuminator can be part of a probe having one or more waveguides. Embodiments can have a collector for collecting light reflected from the sample. Such a collector can encompass a light detector and part of a probe having one or more waveguides such as optical fibers. Embodiments can have a waveguide for coupling the illuminating light to the sample. The waveguide can be of any suitable size or shape depending on the type of sample being measured. It can be arranged so that the illuminating light is diffused in the waveguide to be spread evenly when leaving the outcoupling surface. The outcoupling surface can have a pattern which at least partially surrounds one or more light collection sites used by the collector, and is separated from the collection site or sites.

By using such a waveguide, a greater volume of the sample can be illuminated and the analyte measurements can be made less sensitive to local variations in the sample. This is particularly useful for measurements of samples having complex structures such as cells or tissue or skin for example, which may give widely different reflections from localized variations such as hairs. The spreading of the light so that at the outcoupling surface the light at least partially surrounds a collecting site can help enable a greater volume of the sample which is illuminated to be within a limited range of distances from the collecting site. This can help keep the range of penetration depths better defined, and so improve consistency of analyte measurements.

At least some embodiments can provide a (relatively inexpensive) method of enabling a larger measurement volume with homogenous illumination, using a single or multiple light sources. Some notable features or effects of the embodiments described are: the waveguide to distribute light from one or a small number of light sources over a large area, a suitable spacing of outcoupling sites in a pattern to provide more homogeneous illumination of a greater useful volume of the sample, and a suitable separation to shield collection fibers or photodetectors from the illumination, to enable tighter control of location of the measurement volume.

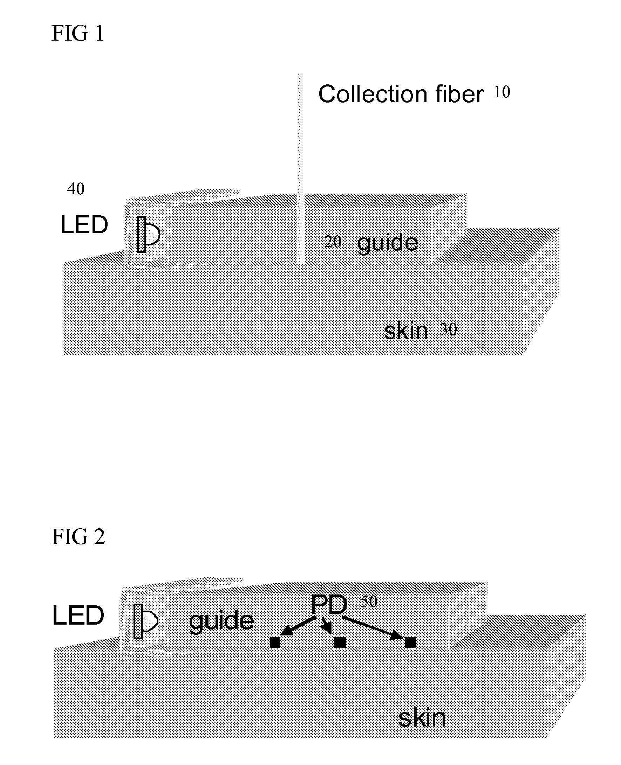

Some additional features are as follows. The apparatus can be arranged so that the illuminating light enters the waveguide from a surface at one side of the outcoupling surface so as to travel laterally across the major surface of the sample and reach the outcoupling surface after one or more reflections. Examples are described below with reference to figures 1-8. The outcoupling surface can cover an area larger than that of the collection sites. The illuminator can comprise two or more light sources, and the waveguide can be arranged to mix and homogenize the light from the two or more sources.

The collector can be distributed, having more than one collecting point spaced apart. The outcoupling surface can have scattering structures to couple out at least some illuminating light incident at an angle which otherwise would be internally reflected. The scattering structures can have outcoupling characteristics which differ at different locations, to compensate for uneven distribution of illuminating light reaching the outcoupling surface.

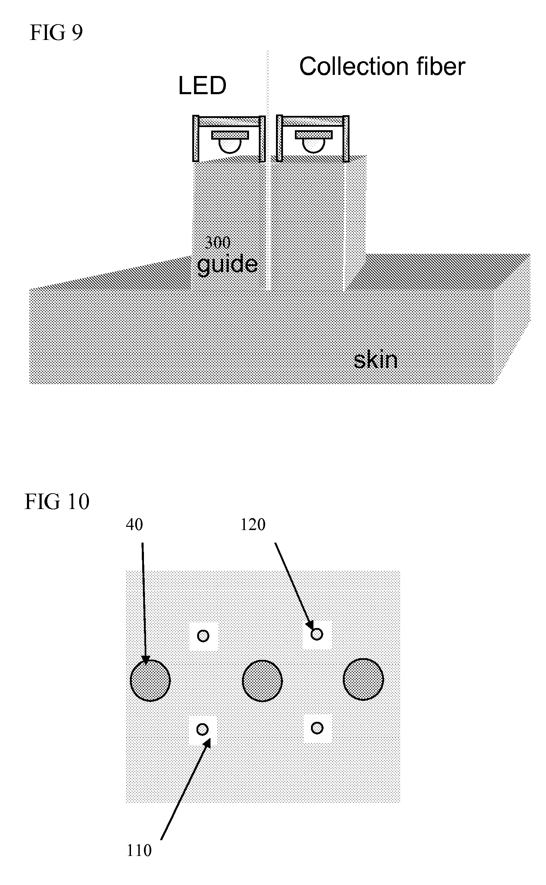

The illuminator can have one or more optical sources facing the outgoing surface. Examples are described below with reference to figures 9 to 11.

Most non-invasive techniques use a small measurement volume when measuring a sample for an analyte. The distance between the launching and collection fiber is not more than 1 - 2 mm. Combinations of multiple small measurement volumes are considered too complicated and are not employed in this case. Using a small measurement volume makes the technique more vulnerable to structural heterogeneity of the skin. The contribution of small interfering structures such as hairs is relatively large. This leads to a high sensitivity of the technique to accurate repositioning. Also for minimally invasive methods the microsensor is usually small in size.

Using a larger measurement volume, the resulting signal would more closely represent the 'average' skin, and accurate repositioning of the probe would be less critical. This can enhance the accuracy, reproducibility and usability of the sensing device. Conventionally, non-homogeneity of illumination is seen as a bar that prevents the use of large measurement volumes. The distinctive features of embodiments of the invention can contribute to a (relatively cheap) way of enabling a larger measurement volume with homogenous illumination, using a single or a small number of light sources.

### Waveguide materials:

Many suitable materials can be envisaged for a solid waveguide. The selection may depend on for example wavelength range, toxicity, solubility, and refractive index. Thermal and mechanical properties of the material may also be important. A chart describing
transmission ranges, refractive index, and other useful information is given in [http://www.internationalcrystal.net/ti_sec1.htm]. A non-toxic, water-resistant material with good transmission between 1000 and 2500 nm is preferred, e.g., CaF₂.

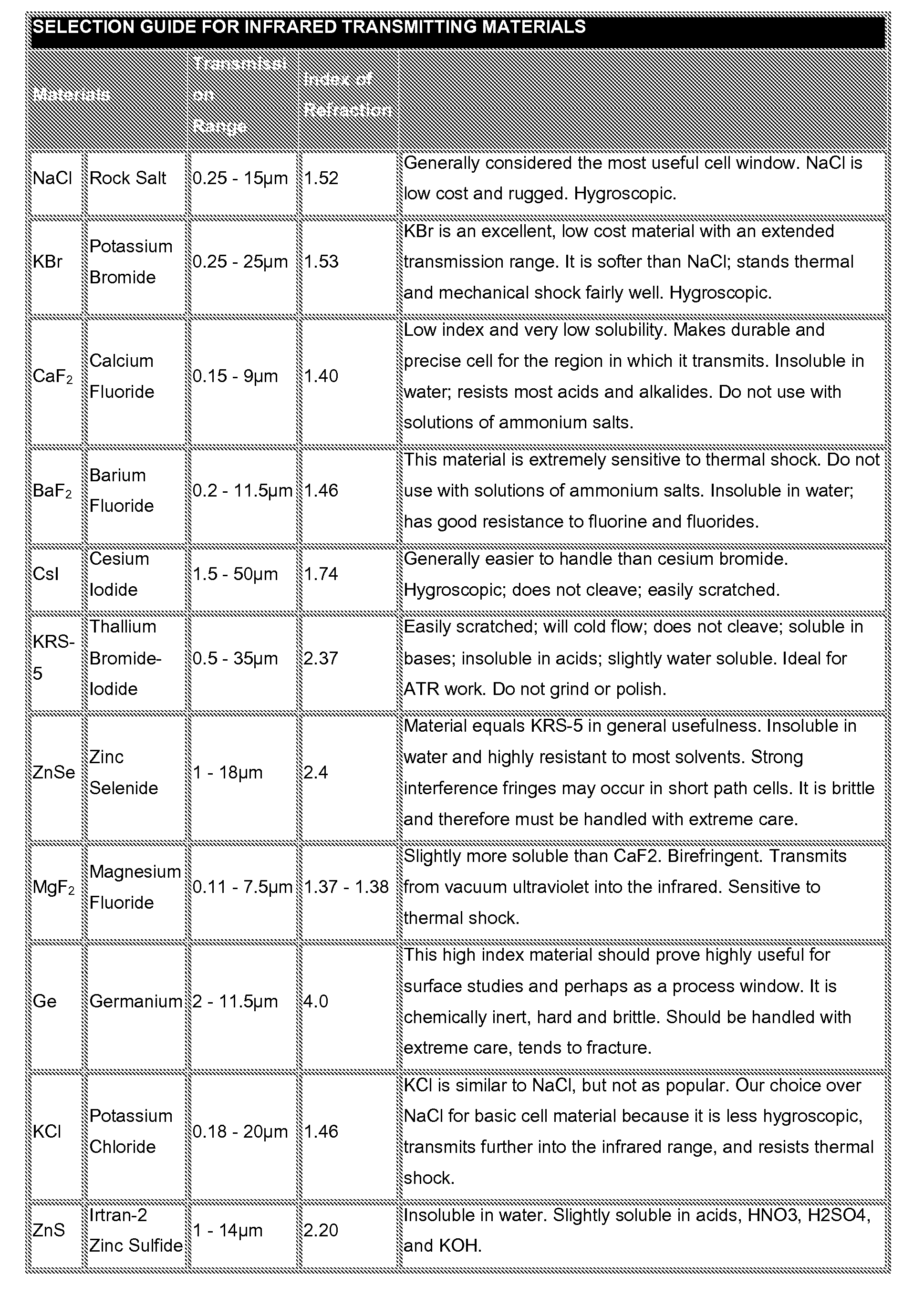

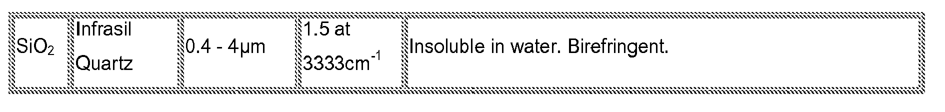

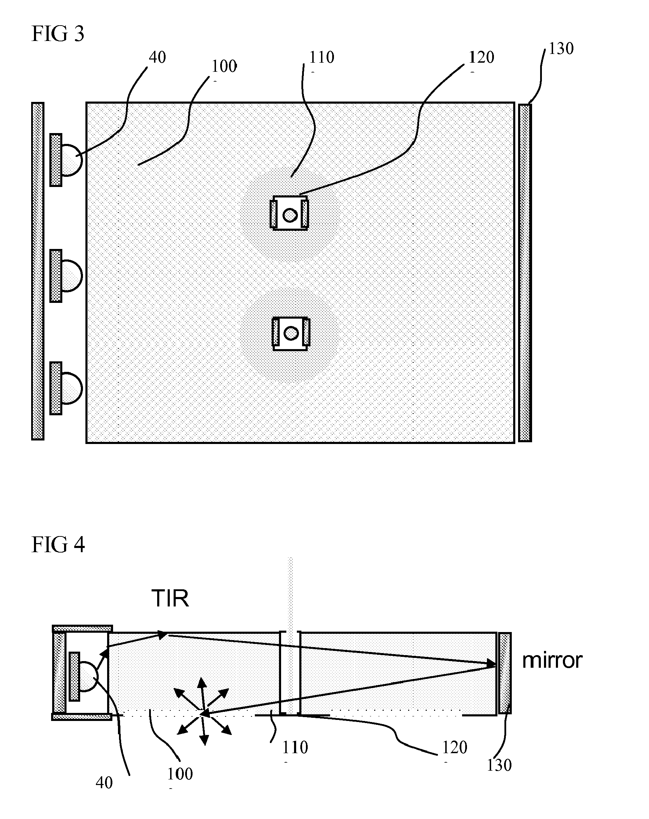

### FIG. 1, 3D perspective view of apparatus according to a first embodiment

FIG. 1 shows a perspective view of a cut through apparatus according to a first embodiment. It includes a waveguide 20 and a side-mounted single light source in the form of an LED 40. The waveguide is placed on the sample in the form of skin 30. The waveguide is largely planar and extends parallel to the surface of the skin. The illuminating light enters the waveguide from the side and is reflected internally which means it is distributed evenly throughout the waveguide. An outcoupling surface is formed by part or parts of a lower surface as shown, adjacent to the skin. This outcoupling surface can be made up of several out coupling locations, where the light is allowed out of the waveguide to enter the skin tissue adjacent. These locations are laid out in a pattern such that the homogeneity of the light distribution is maximized. A collection fiber 10 is used to collect the light that has diffusely reflected back from the skin. The collection fiber meets the skin at a collecting site which is surrounded by the outcoupling surface of the waveguide. The collecting fiber passes through the waveguide, though in principle the optical path for the light collected at the collecting site can go anywhere convenient, to reach a an optical detector to convert the light to an electrical signal. Not shown are the conventional processing parts to process the collected light signal to derive and output measurements to a user.

### FIG. 2 another embodiment using a photo diode

In another embodiment, instead of a collection fiber, a photodetector PD 50 can be used as shown in FIG. 2. This can reduce the complexity of handling of the fiber. Other features are similar to those of FIG. 1 and need not be explained further here.

Around these collection fibers or photodetectors, a blocking layer can be incorporated, to ensure that only light, from skin layers, is collected rather than leakage from the waveguide. Also, the collecting site can be spaced apart from the nearest edge of the outcoupling surface; to ensure that only light from skin layers at the desired penetration depth is collected. In these figures the light source is shown as an LED. Other sources can be used as appropriate to suit the sample. For a minimally invasive or non-invasive glucose monitor a broadband halogen light source can be used. Another option is one or multiple lasers.

### FIGs. 3, 4, embodiment having multiple collectors and light sources

These figures show a plan view and a side cross section view of another embodiment of Fig 3. Three side-mounted LEDs are shown. In principle the side lighting could be on opposing sides, three sides or all four sides for example. A mirror 130 is shown at the far side of the waveguide to maintain the illuminating light in the waveguide so that it undergoes many reflections to achieve better diffusion and mixing. The plan view shows a pattern of the outcoupling surface 100. This extends over most of the bottom surface of the waveguide. Parts 110 of the bottom surface around the collecting site provide a gap to separate the outcoupling surface from the collecting sites 120. Two collecting sites are shown, having an area much smaller than the illuminating area.

Figure 4 shows a side cross section view of the same embodiment. This shows a path of one beam from the light source. It undergoes total internal reflection at the top surface of the waveguide and after reflecting off the mirror 130, it reaches the outcoupling surface 100. This extends on either side of the gap parts 110. The gap parts may be made to be reflective surfaces.

At the outcoupling surface 100, the beam is incident at an angle which would normally cause total internal reflection. But at this surface it is scattered so that a proportion is coupled out into the sample.

The waveguide can achieve diffusion, mixing and spreading by light guiding by Total Internal Reflection (TIR) or by reflectors. The outcoupling can be implemented for example by scattering structures such as dots (for example white paint, sandblasted rough patches, and so on). No outcoupling structure is provided near the collecting site to ensure the collection is only of light which has followed a minimum pathlength through the sample. The implementation of the scattering structures can follow for example practice used for LCD backlight design. For example the scattering dot pitch and/or size can be varied in order to ensure uniform illumination level to compensate for predicted or measured unevenness in distribution or wavelengths of illuminating light reaching the outcoupling surface.

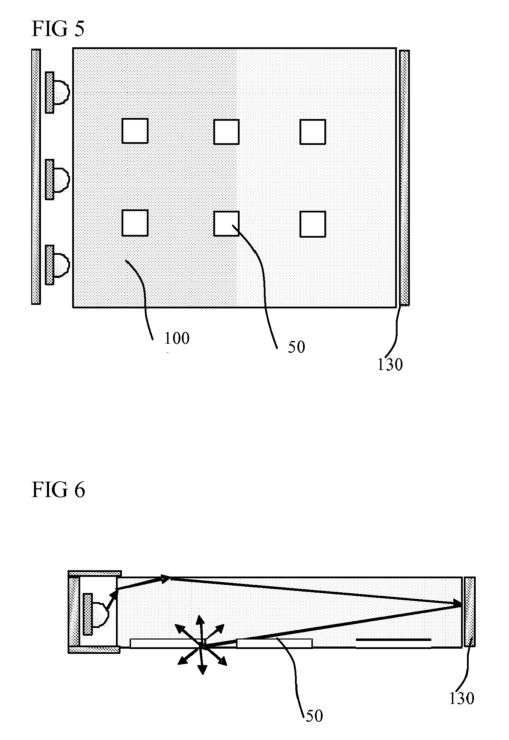

### FIGs. 5, 6, another embodiment using photo diodes

Figures 5 and 6 show a plan view and a side cross section view of another embodiment. Many features are similar to those of figures 3 and 4 and need not be described again. In this case, the collecting sites are shown in the form of six photo diodes 50, distributed across the bottom surface of the waveguide. No collecting fiber is needed. Wires to pass the electrical signals from the photodiodes away from the waveguide can be placed anywhere convenient, and are not shown for the sake of clarity. In between the collecting sites, the outcoupling surface extends everywhere on the bottom surface of the waveguide. A separation part can be implemented in the form of a perimeter or casing of the photodiode. In this way, several narrow beams of light are spread relatively evenly over a large area.

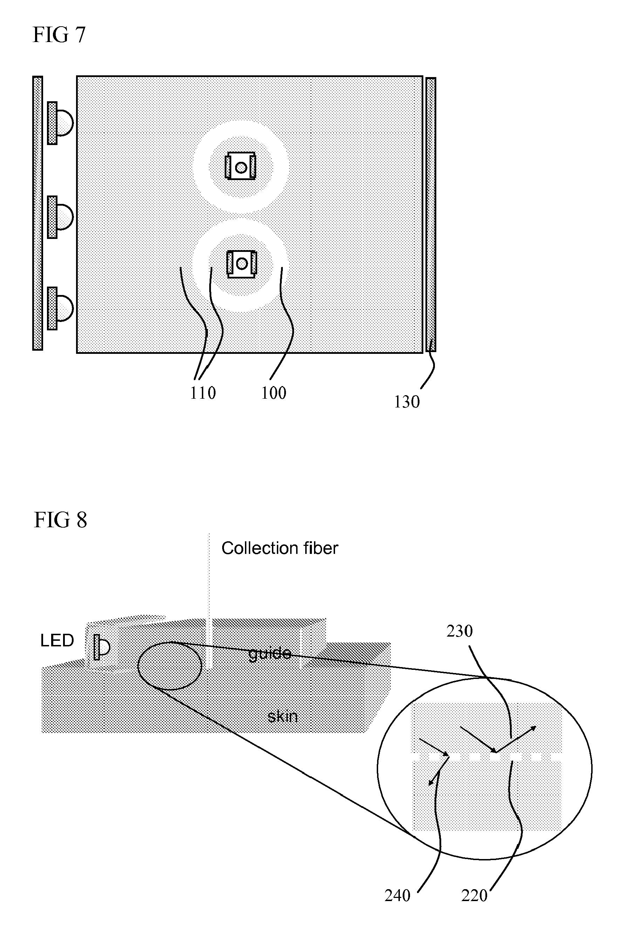

### FIG 7, another embodiment using ring shaped pattern

FIG. 7 shows a plan view of another embodiment. This figure shows an alternative outcoupling surface pattern. A ring shaped pattern 100 of the outcoupling surface is provided around the collecting fiber. This gives a more closely defined range of distances between the illumination and the collecting site on the surface of the sample, which determines the penetration depth in the skin of the collected light. Inside the ring and outside the ring are the gap parts of the bottom surface 110 which provide a gap and are not for outcoupling, and may be reflective. It is not necessary for the ring to surround each collecting site completely; it could extend part of the way round each collecting site.

### FIG 8, another embodiment showing outcoupling surface detail

FIG. 8 shows a 3D perspective view of a cut through apparatus according to another embodiment, with an inset showing scattering at the outcoupling surface. This view shows a similar embodiment to that of figure 1 and reference is made to the description above of similar parts. The inset shows a magnified view of part of the outcoupling surface. Scattering structures in the form of small cubic bumps 220 are shown, spaced apart along the surface. Some light 240 gets through and some light 230 is reflected back into the waveguide. These bumps can help to ensure better TIR by avoiding optical contact with the skin. Outcoupling dots in the form of bumps or spacers create air voids between guide and skin that ensure TIR. Another option for the waveguide is a hollow light guide based on reflectors with holes at one side adjacent to the sample, to let some of the light out, to implement the outcoupling surface.

### FIGs 9, 10, another embodiment showing perpendicular waveguide

FIG. 9 shows a 3D perspective view of a cut through the centre of apparatus according to another embodiment, showing a perpendicular waveguide. FIG. 10 shows a top plan view of the same embodiment. This alternative has a waveguide 300 for light mixing & spreading which is arranged essentially perpendicular to the surface of the skin, in other words it is elongate and has a major axis which extends substantially perpendicular to the skin surface. Two LEDs are shown on the top surface facing the skin, there may be more, spread over the top surface. The waveguide is long enough for reflections from its sides to diffuse and mix the light. A collection fiber is shown passing through the centre of the waveguide. There may be more such fibers spread apart. The outcoupling surface is on the bottom of the waveguide and may extend to the edges of the bottom surface or have a pattern which is not limited by the shape of the bottom surface. The pattern will be arranged to surround the collecting site. As in the laterally arranged waveguide embodiments described above, it can create a uniform illumination over a relatively large, well defined volume of the sample under the outcoupling surface.

In this case there may be less or no issue with skin contact altering the internal reflection characteristics, since the contact is only at the exit window, not at a light guiding surface. The outcoupling surface in this case need not have scattering structures, if enough diffusing takes place by reflections from the sides, to give an even spread of light. Alternatively the scattering structures can be used to provide a more even spreading. The waveguide may be tapered, e.g., with a larger exit than entrance to have a large area and to have more collimated light. The more collimated the light is, the better defined is the measurement volume.

Figure 10 shows a top plan view of this embodiment, showing a number of LEDs 40 spread apart, and a number of collection fibers 120 at collecting sites each having a gap 110 for separating the collecting fiber from the outcoupling surface. In this case the outcoupling surface extends everywhere on the bottom surface between the gaps around each collecting site.

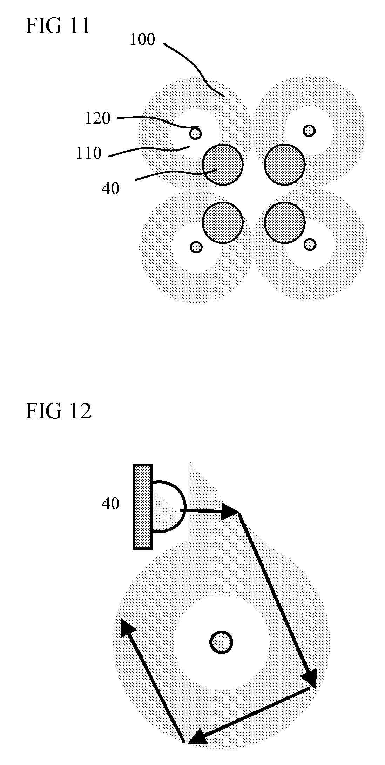

### FIGs 11 and 12, another embodiment.

FIG 11 shows a top view of an alternative arrangement for a perpendicular waveguide. Four LEDs 40 are shown, arranged off centre from their respective ring shaped outcoupling surfaces 100. In this case the waveguide may be formed by 4 cylindrical tubes each surrounding their own collection fiber 120. The pattern of the surface 100 can be seen as a cross section of the cylindrical tube. A separating part 110 around the collecting site is circular in this case, to provide a consistent separation distance.

FIG. 12 shows an alternative illuminating arrangement for the embodiment of figure 11. This is a hybrid arrangement in which the LED is no longer on the top surface of the cylindrical tube, but is on the side of the tube. Light is coupled into the tube in a circumferential direction by a prism. The light spirals downwards which gives a longer ray path and better homogeneity of illumination.

### Application

An opto-mechanical probe skin interface as proposed here, can be used by most techniques that sense analytes within any kind of sample including for example biological samples such as cells, or tissue such as skin. Embodiments of the invention can be used for minimally invasive or non-invasive glucose detection by means of NIR diffuse backreflectance spectroscopy. Further applications include measurements of skin properties (e.g., skin cancer, skin aging, etc.) by means of light.

As has been described above, notable features of at least some embodiments are:

A waveguide to distribute illuminating light over a larger area, using total internal reflection or reflectors.

A suitable spacing of outcoupling sites to ensure homogeneous light distribution.

A suitable configuration of outcoupling sites and collection fibers or photodetectors to control placement of the measurement volume.

Can be used for minimally invasive or non-invasive techniques.

Other variations can be envisaged within the scope of the claims.

## Claims

1. Apparatus for measuring reflectance of a sample, the apparatus having a collector (50, 120) for collecting light reflected from the sample, and a waveguide (20, 100, 300) arranged to couple illuminating light to a surface of the sample through an outcoupling surface (100) of the waveguide, the waveguide being arranged to diffuse the illuminating light so that light leaving the outcoupling surface is evenly spread, and the outcoupling surface has a pattern which at least partially surrounds one or more light collection sites used by the collector, and is separated from the collection site or sites.

2. The apparatus of claim 1, arranged so that the illuminating light enters the waveguide from a surface at one side of the outcoupling surface so as to travel laterally across the major surface and reach the outcoupling surface after one or more reflections.

3. The apparatus of claim 1 or 2, the outcoupling surface covering an area larger than that of the collection sites.

4. The apparatus of any preceding claim, having two or more light sources, and the waveguide being arranged to mix and homogenize the light from the two or more sources.

5. The apparatus of any preceding claim, the collector being distributed, having more than one collecting point spaced apart.

6. The apparatus of any preceding claim, the outcoupling surface having scattering structures (220) to couple out at least some illuminating light incident at an angle which otherwise be internally reflected.

7. The apparatus of claim 6, the scattering structures having outcoupling characteristics which differ at different locations, to compensate for uneven distribution of illuminating light reaching the outcoupling surface.

8. The apparatus of any preceding claim, having one or more optical sources facing the outcoupling surface.

9. The apparatus of any preceding claim suitable for minimally invasive or non invasive glucose measurements on skin.

10. A method of measuring reflectance of a sample, the method having the steps of using a waveguide (20, 100, 300) to couple illuminating light to a surface of the sample through an outcoupling surface (100) of the waveguide, the waveguide being arranged to diffuse the illuminating light so that light leaving the outcoupling surface is evenly spread, and the outcoupling surface has a pattern which at least partially surrounds one or more light collection sites, and is separated from the collection site or sites, and the step of collecting light reflected from the sample, at one or more light collection sites.

11. The method of claim 10, the waveguide being arranged so that the illuminating light enters the waveguide from a surface at one side of the outcoupling surface so as to travel laterally across the major surface and reach the outcoupling surface after one or more reflections.

12. The method of any preceding claim, the measuring being measuring of glucose in skin.

13. A probe for measuring reflectance of a sample, the probe having a waveguide (20, 100, 300) arranged to couple illuminating light to a surface of the sample through an outcoupling surface (100) of the waveguide, the waveguide being arranged to diffuse the illuminating light so that light leaving the outcoupling surface is evenly spread, and the outcoupling surface has a pattern which at least partially surrounds one or more light collection sites used for measuring the reflectance, and is separated from the collection site or sites.
